(19) Europäisches Patentamt
European Patent Office
Office européen des brevets

(11) **EP 4 215 273 A1**

(12) **EUROPEAN PATENT APPLICATION**
published in accordance with Art. 153(4) EPC

(43) Date of publication:
**26.07.2023 Bulletin 2023/30**

(21) Application number: **21869199.6**

(22) Date of filing: **03.09.2021**

(51) International Patent Classification (IPC):
*B01J 35/02* (2006.01)    *A61L 9/00* (2006.01)
*A61L 9/01* (2006.01)    *B01J 21/06* (2006.01)
*B01J 23/847* (2006.01)    *B01J 23/887* (2006.01)
*B01J 23/888* (2006.01)    *B01J 37/04* (2006.01)
*B01J 37/10* (2006.01)

(52) Cooperative Patent Classification (CPC):
**A61L 9/00; A61L 9/01; B01J 21/06; B01J 23/847;
B01J 23/887; B01J 23/888; B01J 35/02;
B01J 37/04; B01J 37/10**

(86) International application number:
**PCT/JP2021/032391**

(87) International publication number:
**WO 2022/059512 (24.03.2022 Gazette 2022/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **15.09.2020 JP 2020154252**

(71) Applicant: **SHIN-ETSU CHEMICAL CO., LTD.
Tokyo 1000005 (JP)**

(72) Inventors:
• FURUDATE Manabu
  Kamisu-shi, Ibaraki 314-0102 (JP)
• INOUE Tomohiro
  Kamisu-shi, Ibaraki 314-0102 (JP)
• HINOUE Mikiya
  Kamisu-shi, Ibaraki 314-0102 (JP)

(74) Representative: **Angerhausen, Christoph
Boehmert & Boehmert
Anwaltspartnerschaft mbB
Pettenkoferstrasse 22
80336 München (DE)**

(54) **TITANIUM OXIDE PARTICLES, DISPERSION LIQUID OF SAME, PHOTOCATALYST THIN FILM, MEMBER HAVING PHOTOCATALYST THIN FILM ON SURFACE, AND METHOD FOR PRODUCING DISPERSION LIQUID OF TITANIUM OXIDE PARTICLES**

(57)    Provided are titanium oxide particles having a higher photocatalytic activity, particularly a higher visible light activity as compared to the conventional ones; a dispersion liquid thereof; a photocatalyst thin film formed using such dispersion liquid; a member having such photocatalyst thin film on its surface; and a method for producing the titanium oxide particle dispersion liquid. The titanium oxide particles are those with (1) a tin component and a visible light activity-enhancing transition metal component being solid-dissolved in the particles; and with (2) an iron component, a titanium component and a silicon component being adhered to the surfaces of the particles. The titanium oxide particle dispersion liquid is one with the titanium oxide particles being dispersed in an aqueous dispersion medium.

EP 4 215 273 A1

**Description**

TECHNICAL FIELD

The present invention relates to titanium oxide particles, a dispersion liquid thereof, a photocatalyst thin film formed using such dispersion liquid, a member having the photocatalyst thin film on surface, and a method for producing a titanium oxide particle dispersion liquid; more specifically relates to, for example, visible light responsive photocatalyst titanium oxide particles capable of expressing a photocatalytic activity even under only a visible light (wavelength 400 to 800 nm), and being used to easily produce a photocatalyst thin film having a high transparency.

BACKGROUND ART

Photocatalysts are often used for the purposes of, for example, cleaning, deodorizing and bringing about an antibacterial effect on the surface of a member. A photocatalytic reaction refers to a reaction caused by excited electrons and positive holes that have occurred as a result of having a photocatalyst absorb a light. It is considered that the decomposition of an organic substance by a photocatalyst is mainly triggered by the following mechanisms [1] or [2].

[1] The excited electrons and positive holes that have been generated undergo a redox reaction with the oxygen and water that have adsorbed to the surface of the photocatalyst, so that one or more active species that have occurred due to the redox reaction shall decompose the organic substance.
[2] The positive holes that have been generated decompose the organic substance that have adsorbed to the surface of the photocatalyst by directly oxidizing the same.

Recently, as for the application of the above photocatalytic action, studies are being conducted on uses not only outdoors where ultraviolet light is available, but also indoors where a light source(s) mostly composed of lights in the visible region (wavelength 400 to 800 nm), such as a fluorescent light, are used for illumination. For example, as a visible light responsive photocatalyst, there has been developed a tungsten oxide photocatalyst body (JP-A-2009-148700: Patent document 1); since tungsten is a scarce element, it is desired that titanium as a general element be utilized to enhance the visible light activity of a photocatalyst.

As a method for enhancing the visible light activity of a photocatalyst utilizing titanium oxide, there are known, for example, a method of having iron and/or copper supported on the surfaces of titanium oxide fine particles and titanium oxide fine particles doped with a metal (e.g. JP-A-2012-210632: Patent document 2, JP-A-2010-104913: Patent document 3, JP-A-2011-240247: Patent document 4, JP-A-Hei-7-303835: Patent document 5); a method where there are at first separately prepared titanium oxide fine particles with tin and a visible light activity-enhancing transition metal solid-dissolved (doped) therein and titanium oxide fine particles with copper solid-dissolved therein, followed by mixing them before use (WO2014/045861: Patent document 6); and a method where there are at first separately prepared titanium oxide fine particles with tin and a visible light responsiveness-enhancing transition metal solid-dissolved therein and titanium oxide fine particles with an iron group element solid-dissolved therein, followed by mixing them before use (WO2016/152487: Patent document 7).

As a result of employing a photocatalyst film formed using a visible light-responsive photocatalyst titanium oxide fine particle dispersion liquid that is obtained by mixing the separately prepared titanium oxide fine particles with tin and a visible light activity-enhancing transition metal solid-dissolved therein and the separately prepared titanium oxide fine particles with an iron group element solid-dissolved therein as is the case with Patent document 7, a high decomposition activity can be achieved under a condition where only lights in the visible region are available. Further, it has been demonstrated that an acetaldehyde gas can be likewise decomposed under a condition where only lights in the visible region are available even when employing a photocatalyst thin film formed using a titanium oxide fine particle dispersion liquid in which an iron component is adsorbed to (=supported on) the surfaces of titanium oxide fine particles with tin and a visible light activity-enhancing transition metal being solid-dissolved therein; however, a low photocatalytic activity was observed as a result of restricting the amount of the iron component due to the fact that the quality of the photocatalyst film obtained will be impaired as the titanium oxide fine particles agglutinate and precipitate because of the iron component.

As described above, although a number of studies are being conducted to improve photocatalytic activity, further improvements in photocatalytic activity are demanded as it is critical to decompose and eliminate hazardous substances as rapidly as possible in the actual environment.

PRIOR ART DOCUMENTS

Patent documents

Patent document 1: JP-A-2009-148700
Patent document 2: JP-A-2012-210632
Patent document 3: JP-A-2010-104913
Patent document 4: JP-A-2011-240247
Patent document 5: JP-A-Hei-7-303835
Patent document 6: WO2014/045861
Patent document 7: WO2016/152487

SUMMARY OF THE INVENTION

Problems to be solved by the invention

Thus, it is an object of the present invention to provide titanium oxide particles with which there can be achieved an even higher photocatalytic activity, particularly a higher visible light activity as compared to the conventional ones; a dispersion liquid thereof; a photocatalyst thin film formed using such dispersion liquid; a member having such photocatalyst thin film on its surface; and a method for producing the titanium oxide particle dispersion liquid.

Means to solve the problems

In order to achieve the above object, the inventors of the present invention completed the invention as follows. That is, as a result of precisely studying, for example, metal elements to be solid-dissolved in titanium oxide particles and combinations thereof, metal elements to be added to titanium oxide particles and combinations thereof, and quantitative ratios thereof, the inventors noticed a dramatic improvement in photocatalytic activity, particularly visible light activity in the case of titanium oxide particles with a particular metal(s) solid-dissolved therein and with an iron, a titanium and a silicon component adhered to the surfaces thereof.

Thus, the present invention is to provide titanium oxide particles described below; a dispersion liquid thereof; a photocatalyst thin film formed using such dispersion liquid; a member having such photocatalyst thin film on its surface; and a method for producing the titanium oxide particle dispersion liquid.

[1] Titanium oxide particles with

(1) a tin component and a visible light activity-enhancing transition metal component being solid-dissolved in the particles; and with
(2) an iron component, a titanium component and a silicon component being adhered to the surfaces of the particles.

[2] The titanium oxide particles according to [1], wherein the visible light activity-enhancing transition metal component solid-dissolved in the titanium oxide particles is at least one selected from vanadium, chromium, manganese, niobium, molybdenum, rhodium, tungsten and cerium.

[3] The titanium oxide particles according to [2], wherein the visible light activity-enhancing transition metal component solid-dissolved in the titanium oxide particles is at least one selected from a molybdenum component, a tungsten component and a vanadium component.

[4] The titanium oxide particles according to any one of [1] to [3], wherein the tin component is contained and solid-dissolved in the titanium oxide particles by an amount of 1 to 1,000 in terms of a molar ratio to titanium oxide ($TiO_z/Sn$).

[5] The titanium oxide particles according to any one of [1] to [4], wherein a molar ratio of the iron component to titanium oxide ($TiO_2/Fe$) is 10 to 10,000, a molar ratio of the titanium component to titanium oxide ($TiO_z/Ti$) is 10 to 10,000, and a molar ratio of the silicon component to titanium oxide ($TiO_2/Si$) is 1 to 10,000.

[6] The titanium oxide particles according to [3], wherein the molybdenum, tungsten and vanadium components solid-dissolved in the titanium oxide particles are each in an amount of 1 to 10,000 in terms of a molar ratio to titanium oxide ($TiO_2/Mo$, $TiO_2/W$ or $TiO_2/V$).

[7] A titanium oxide particle dispersion liquid with the titanium oxide particles according to any one of [1] to [6] being dispersed in an aqueous dispersion medium.

[8] The titanium oxide particle dispersion liquid according to [7], wherein the titanium oxide particle dispersion liquid further comprises a binder.

[9] The titanium oxide particle dispersion liquid according to [8], wherein the binder is a silicon compound-based binder.

[10] A photocatalyst thin film comprising the titanium oxide particles according to any one of [1] to [6].

[11] The photocatalyst thin film according to [10], wherein the photocatalyst thin film further comprises a binder.

[12] A member having, on a surface thereof, the photocatalyst thin film according to [10] or [11].

[13] A method for producing the titanium oxide particle dispersion liquid according to any one of [7] to [9], comprising:

(1) a step of producing a tin component and transition metal component-containing peroxotitanic acid solution from a raw material titanium compound, tin compound, transition metal compound, basic substance, hydrogen peroxide and aqueous dispersion medium;

(2) a step of obtaining a tin component and transition metal component-containing titanium oxide particle dispersion liquid by heating the tin component and transition metal component-containing peroxotitanic acid solution produced in the step (1) at 80 to 250°C under a controlled pressure;

(3) a step of producing a solution or dispersion liquid of the iron component, titanium component and silicon component from an iron compound, titanium compound, silicon compound and aqueous dispersion medium; and

(4) a step of obtaining a dispersion liquid by mixing the titanium oxide particle dispersion liquid that has been produced in the step (2) and the solution or dispersion liquid of the iron, titanium and silicon compounds that has been produced in the step (3).

Effects of the invention

The titanium oxide particles of the present invention have an even higher photocatalytic activity as compared to the conventional ones, and particularly have a high photocatalytic activity even when only visible lights (wavelength 400 to 800 nm) are available. Further, a highly transparent photocatalyst thin film can be easily produced from the dispersion liquid of such titanium oxide particles. Thus, the titanium oxide particles of the present invention are suitable for use in members that are used indoors where a light source(s) mostly composed of visible lights, such as a fluorescent light and a white LED, are used for illumination.

MODE FOR CARRYING OUT THE INVENTION

The present invention is described in detail hereunder.

<Titanium oxide particle dispersion liquid>

A titanium oxide particle dispersion liquid of the present invention is one containing, in an aqueous dispersion medium, (1) titanium oxide particles with a tin component and a visible light activity-enhancing transition metal solid-dissolved therein; and (2) an iron component, a titanium component and a silicon component. The iron, titanium and silicon components contained in the titanium oxide particle dispersion liquid are those adhering to the surfaces of the titanium oxide particles; the iron, titanium and silicon components may also be free in the titanium oxide particle dispersion liquid.

As the aqueous dispersion medium, while water is preferably used, there may also be used a mixed solvent of water and a hydrophilic organic solvent which is to be mixed with water at any ratio. As water, preferred are, for example, purified waters such as a filtrate water, a deionized water, a distilled water and a pure water. Moreover, as the hydrophilic organic solvent, preferred are, for example, alcohols such as methanol, ethanol and isopropanol; glycols such as ethylene glycol; and glycol ethers such as ethylene glycol monomethyl ether, ethylene glycol monoethyl ether and propylene glycol-n-propyl ether. If using such mixed solvent, it is preferred that a ratio of the hydrophilic organic solvent in the mixed solvent be larger than 0% by mass, but not larger than 50% by mass; more preferably larger than 0% by mass, but not larger than 20% by mass; even more preferably larger than 0% by mass, but not larger than 10% by mass.

The titanium oxide particles are those with the tin component and the visible light activity-enhancing transition metal component solid-dissolved in titanium oxide used as a photocatalyst; the expression that the titanium oxide particle dispersion liquid of the present invention "contains the iron component, the titanium component and the silicon component" means that these iron, titanium and silicon components are contained in the dispersion liquid.

As a crystal phase of titanium oxide particles, there are generally known three of them which are the rutile-type, anatase-type and brookite-type. It is preferred that the titanium oxide particles of the present invention mainly be the rutile-type or anatase-type, particularly preferably the rutile-type. Here, the expression "mainly" refers to a condition where the titanium oxide particles having such particular crystal phase(s) are contained in the titanium oxide particles as a whole by an amount of not smaller than 50% by mass, preferably not smaller than 70% by mass, even more preferably not smaller than 90% by mass, or even 100% by mass.

Here, in this specification, a solid solution refers to that having a phase where atoms at lattice points in a certain crystal phase have been substituted by other atoms or where other atoms have entered lattice spacings i.e. a mixed phase regarded as one with a different substance(s) dissolved into a certain crystal phase, and being a homogeneous phase as a crystal phase. A solid solution where solvent atoms at lattice points have been substituted by solute atoms is called a substitutional solid solution, and a solid solution where solute atoms have entered lattice spacings is called an interstitial solid solution; in this specification, a solid solution refers to both of them.

The titanium oxide particles of the present invention are characterized by forming a solid solution with tin atoms and visible light responsiveness-enhancing transition metal atoms. The solid solution may be either substitutional or interstitial. A substitutional solid solution of titanium oxide is formed by having titanium sites of a titanium oxide crystal substituted by various metal atoms; an interstitial solid solution of titanium oxide is formed by having various metal atoms enter the lattice spacings of a titanium oxide crystal. After various metal atoms have been solid-dissolved into titanium oxide, when measuring the crystal phase by X-ray diffraction or the like, there will only be observed the peak of the crystal phase of titanium oxide, whereas there will not be observed peaks of compounds derived from various metal atoms added.

While there are no particular restrictions on a method for solid-dissolving dissimilar metals into a metal oxide crystal, there may be listed, for example, a gas phase method (e.g. CVD method, PVD method), a liquid phase method (e.g. hydrothermal method, sol-gel process) and a solid phase method (e.g. high-temperature firing).

While the tin component to be solid-dissolved in the titanium oxide particles is for enhancing the visible light responsiveness of a photocatalyst thin film, it will suffice if the tin component is that derived from a tin compound, examples of which include elemental tin as a metal (Sn), a tin oxide (SnO, $SnO_z$), a tin hydroxide, a tin chloride ($SnCl_z$, $SnCl_4$), a tin nitrate ($Sn(NO_3)_2$), a tin sulfate ($SnSO_4$), a tin halide (Br, I) other than a tin chloride, a salt of tin-oxoacid (stannate) ($Na_2SnO_3$, $K_2SnO_3$) and a tin complex compound; there may be used one of them or a combination of two or more of them. Particularly, it is preferred that there be used a tin oxide (SnO, $SnO_z$), a tin chloride ($SnCl_z$, $SnCl_4$), a tin sulfate ($SnSO_4$) and a salt of tinoxoacid (stannate) ($Na_2SnO_3$, $K_2SnO_3$).

The tin component is solid-dissolved in the titanium oxide particles by an amount of 1 to 1,000, preferably 5 to 500, more preferably 5 to 100, in terms of a molar ratio to titanium oxide ($TiO_z$/Sn). This is because if the molar ratio is lower than 1, a photocatalytic effect may not be sufficiently exhibited as titanium oxide is now contained at a lower rate; and if the molar ratio is greater than 1,000, an insufficient visible light responsiveness may be observed.

The transition metal to be solid-dissolved in the titanium oxide particles is for enhancing the visible light responsiveness of the photocatalyst thin film; the transition metal is one or more elements selected from the group 3 to group 11 in the periodic table, and may be selected from vanadium, chromium, manganese, niobium, molybdenum, rhodium, tungsten, cerium and the like, among which molybdenum, tungsten and vanadium are preferred.

The transition metal component to be solid-dissolved in the titanium oxide particles may be that derived from a corresponding transition metal compound, examples of which include a metal, an oxide, a hydroxide, a chloride, a nitrate, a sulfate, a halide (Br, I) other than a chloride, a salt of oxoacid and various complex compounds; there may be used one of them or a combination of two or more of them.

The amount of the transition metal component(s) to be solid-dissolved in the titanium oxide particles may be appropriately determined based on the type of the transition metal component; it is preferred that the amount thereof be 1 to 10,000 in terms of a molar ratio to titanium oxide ($TiO_z$/transition metal).

When molybdenum is selected as the transition metal component to be solid-dissolved in the titanium oxide particles, it will suffice if the molybdenum component is that derived from a molybdenum compound, examples of which include elemental molybdenum as a metal (Mo), a molybdenum oxide ($MoO_2$, $MoO_s$), a molybdenum hydroxide, a molybdenum chloride ($MoCl_3$, $MoCl_s$), a molybdenum nitrate, a molybdenum sulfate, a molybdenum halide (Br, I) other than a molybdenum chloride, a molybdic acid (molybdenum-oxoacid) and a salt thereof ($H_2MoO_4$, $Na_2MoO_4$, $K_2MoO_4$), and a molybdenum complex compound; there may be used one of them or a combination of two or more of them. Particularly, it is preferred that there be used a molybdenum oxide ($MoO_z$, $MoO_s$), a molybdenum chloride ($MoCl_3$, $MoCl_s$), and a molybdenum-oxoacid and a salt thereof ($H_2MoO_4$, $Na_2MoO_4$, $K_2MoO_4$).

The molybdenum component is preferably solid-dissolved in the titanium oxide particles by an amount of 1 to 10,000, more preferably 5 to 5,000, even more preferably 20 to 1,000, in terms of a molar ratio to titanium oxide ($TiO_2$/Mo). This is because if the molar ratio is lower than 1, a photocatalytic effect may not be sufficiently exhibited as titanium oxide is now contained at a lower rate; and if the molar ratio is greater than 10,000, an insufficient visible light responsiveness may be observed.

When tungsten is selected as the transition metal component to be solid-dissolved in the titanium oxide particles, it will suffice if the tungsten component is that derived from a tungsten compound, examples of which include elemental tungsten as a metal (W), a tungsten oxide ($WO_3$), a tungsten hydroxide, a tungsten chloride ($WCl_4$, $WCl_6$), a tungsten

nitrate, a tungsten sulfate, a tungsten halide (Br, I) other than a tungsten chloride, a tungstic acid and salt of tungsten-oxoacid (tungstate) ($H_2WO_4$, $Na_2WO_4$, $K_2WO_4$), and a tungsten complex compound; there may be used one of them or a combination of two or more of them. Particularly, it is preferred that there be used a tungsten oxide ($WO_3$), a tungsten chloride ($WCl_4$, $WCl_6$) and a salt of tungsten-oxoacid (tungstate) ($Na_2WO_4$, $K_2WO_4$).

The tungsten component is preferably solid-dissolved in the titanium oxide particles by an amount of 1 to 10,000, more preferably 5 to 5,000, even more preferably 20 to 2,000, in terms of a molar ratio to titanium oxide ($TiO_2$/W). This is because if the molar ratio is lower than 1, a photocatalytic effect may not be sufficiently exhibited as titanium oxide is now contained at a lower rate; and if the molar ratio is greater than 10,000, an insufficient visible light responsiveness may be observed.

When vanadium is selected as the transition metal component to be solid-dissolved in the titanium oxide particles, it will suffice if the vanadium component is that derived from a vanadium compound, examples of which include elemental vanadium as a metal (V), a vanadium oxide (VO, $V_2O_3$, VOz, $V_2O_5$), a vanadium hydroxide, a vanadium chloride ($VCl_5$), a vanadium oxychloride ($VOCl_3$), a vanadium nitrate, a vanadium sulfate, a vanadyl sulfate ($VOSO_4$), a vanadium halide (Br, I) other than a vanadium chloride, a salt of vanadium-oxoacid (vanadate) ($Na_3VO_4$, $K_3VO_4$, $KVO_3$), and a vanadium complex compound; there may be used one of them or a combination of two or more of them. Particularly, it is preferred that there be used a vanadium oxide ($V_2O_3$, $V_2O_5$), a vanadium chloride ($VCl_5$), a vanadium oxychloride ($VOCl_3$), a vanadyl sulfate ($VOSO_4$), and a salt of vanadium-oxoacid (vanadate) ($Na_3VO_4$, $K_3VO_4$, $KVO_3$).

The vanadium component is preferably solid-dissolved in the titanium oxide particles by an amount of 1 to 10,000, more preferably 10 to 10,000, even more preferably 100 to 10,000, in terms of a molar ratio to titanium oxide ($TiO_2$/V). This is because if the molar ratio is lower than 1, a photocatalytic effect may not be sufficiently exhibited as titanium oxide is now contained at a lower rate; and if the molar ratio is greater than 10,000, an insufficient visible light responsiveness may be observed.

As the transition metal component(s) to be solid-dissolved in the titanium oxide particles, there may also be selected multiple components from molybdenum, tungsten and vanadium. The amount of each component at that time may be selected from the above ranges. However, a molar ratio between a sum of the components and titanium oxide [$TiO_2$/(Mo+W+V)] is not lower than 1, but lower than 10,000.

As the titanium oxide particles, one kind thereof may be used, or two or more kinds thereof may be used in combination. There may be achieved an effect of enhancing a visible light activity if combining two or more kinds of the titanium oxide particles having different visible light responsivenesses.

The iron, titanium and silicon components contained in the titanium oxide particle dispersion liquid and adhering to the surfaces of the titanium oxide particles are for enhancing the visible light responsiveness of the photocatalyst thin film.

While the iron component contained in the titanium oxide particle dispersion liquid is for enhancing the photocatalytic activity of the photocatalyst thin film, it will suffice if the iron component is that derived from an iron compound, examples of which include elemental iron as a metal (Fe), an iron oxide ($Fe_2O_3$, $Fe_3O_4$), an iron hydroxide ($Fe(OH)_2$, $Fe(OH)_3$), an iron oxyhydroxide (FeO(OH)), an iron chloride ($FeCl_2$, $FeCl_3$), an iron nitrate ($Fe(NO)_3$), an iron sulfate ($FeSO_4$, $Fe_2(SO_4)_3$), an iron halide (Br, I) other than an iron chloride, and an iron complex compound; there may be used one of them or a combination of two or more of them.

The iron component is preferably contained in the titanium oxide particle dispersion liquid by an amount of 10 to 10,000, more preferably 20 to 5,000, even more preferably 50 to 2,000, in terms of a molar ratio to titanium oxide ($TiO_2$/Fe). This is because if the molar ratio is lower than 10, the titanium oxide will agglutinate and precipitate so that the quality of the photocatalyst thin film obtained will deteriorate, and the photocatalytic effect thereof may thus not be exerted in a sufficient manner; and if the molar ratio is greater than 10,000, an insufficient visible light responsiveness may be observed.

While the titanium component contained in the titanium oxide particle dispersion liquid is for enhancing the photocatalytic activity of the photocatalyst thin film, it will suffice if the titanium component is that derived from a titanium compound, examples of which include elemental titanium as a metal (Ti), a titanium hydroxide ($Ti(OH)_4$), a titanium oxyhydroxide ($TiO(OH)_2$), a titanium chloride ($TiCl_4$, $TiCl_3$, TiClz), a titanium nitrate ($Ti(NO)_4$), a titanium sulfate ($Ti(SO_4)_2$, $TiOSO_4$), a titanium halide (Br, I) other than a titanium chloride, and a titanium complex compound; there may be used one of them or a combination of two or more of them.

The titanium component is preferably contained in the titanium oxide particle dispersion liquid by an amount of 10 to 10,000, more preferably 20 to 5,000, even more preferably 50 to 2,000, in terms of a molar ratio to titanium oxide (TiOz/Ti). This is because if the molar ratio is lower than 10, titanium oxide will agglutinate and precipitate so that the quality of the photocatalyst thin film obtained will deteriorate, and the photocatalytic effect thereof may thus not be exerted in a sufficient manner; and if the molar ratio is greater than 10,000, there may be observed an insufficient effect of enhancing the activity.

The silicon component contained in the titanium oxide particle dispersion liquid is for inhibiting deterioration in photocatalytic effect by avoiding deterioration in quality of the photocatalyst thin film as a result of inhibiting the agglutination and precipitation of titanium oxide as well as the iron and titanium components at the time of adding the iron and titanium components. It will suffice if the silicon component is that derived from a silicon compound, examples of which include elemental silicon as a metal (Si), a silicon oxide (SiO, SiOz), a silicon alkoxide (Si(OCH$_3$)$_4$, Si(OC$_2$H$_5$)$_4$, Si(OCH(CH$_3$)$_2$)$_4$), a silicate (sodium silicate, potassium silicate), and an active silicate obtained by removing at least part of ions such as sodium ions and potassium ions from such silicate; there may be used one of them or a combination of two or more of them. Among them, it is preferred that there be used a silicate (sodium silicate) and an active silicate, particularly preferably an active silicate.

The silicon component is preferably contained in the titanium oxide particle dispersion liquid by an amount of 1 to 10,000, more preferably 2 to 5,000, even more preferably 5 to 2,000, in terms of a molar ratio to titanium oxide (TiOz/Si). This is because if the molar ratio is lower than 1, the photocatalytic effect may not be sufficiently exhibited as titanium oxide is now contained at a lower rate; and if the molar ratio is greater than 10,000, there may be observed an insufficient effect of inhibiting the agglutination and precipitation of titanium oxide.

It is preferred that the titanium oxide particles in the titanium oxide particle dispersion liquid containing the iron, titanium and silicon components each have a particle diameter of 3 to 50 nm, more preferably 3 to 30 nm, even more preferably 3 to 20 nm, the particle diameter being a 50% cumulative distribution diameter (possibly referred to as D$_{50}$ hereunder) on volumetric basis that is measured by a dynamic light scattering method using a laser light. This is because if D$_{50}$ is smaller than 3 nm, an insufficient photocatalytic activity may be observed; and if D$_{50}$ is greater than 50 nm, the dispersion liquid may be opaque.

Further, as for a 90% cumulative distribution diameter (possibly referred to as D$_{90}$ hereunder) on volumetric basis of the titanium oxide particles, it is preferred that such diameter be 5 to 100 nm, more preferably 5 to 80 nm. This is because if D$_{90}$ is smaller than 5 nm, an insufficient photocatalytic activity may be observed; and if D$_{90}$ is greater than 100 nm, the dispersion liquid may be opaque.

It is preferred that the titanium oxide particles of the present invention are particles whose D$_{50}$ and D$_{90}$ are within the above ranges, because there can be achieved a dispersion liquid with a high photocatalytic activity and a high transparency.

Here, as a device for measuring D$_{50}$ and D$_{90}$ of the titanium oxide particles in the titanium oxide particle dispersion liquid, there may be used, for example, ELSZ-2000ZS (by Otsuka Electronics Co., Ltd.), NANOTRAC UPA-EX150 (by Nikkiso Co., Ltd.) or LA-910 (by HORIBA, Ltd.).

It is preferred that a concentration of the titanium oxide particles in the titanium oxide particle dispersion liquid be 0.01 to 20% by mass, particularly preferably 0.5 to 10% by mass, in terms of ease in producing a photocatalyst thin film having a given thickness.

Further, a binder may also be added to the titanium oxide particle dispersion liquid for the purpose of making it easy to apply the dispersion liquid to the surfaces of later-described various members, and allow the particles to adhere thereto. Examples of the binder include metal compound-based binders containing silicon, aluminum, titanium, zirconium or the like; and organic resin-based binders containing an acrylic resin, a urethane resin or the like.

It is preferred that the binder be added and used in a manner such that a mass ratio between the binder and titanium oxide [titanium oxide/binder] will fall into a range of 99 to 0.01, more preferably 9 to 0.1, even more preferably 2.5 to 0.4. This is because if the mass ratio is greater than 99, the titanium oxide particles may adhere to the surfaces of various members in an insufficient manner; and if the mass ratio is lower than 0.01, an insufficient visible light activity may be observed.

Particularly, in order to obtain an excellent photocatalyst thin film having a high photocatalytic activity and transparency, it is preferred that a silicon compound-based binder be added and used in a manner such that the mass ratio (titanium oxide/silicon compound-based binder) will fall into the range of 99 to 0.01, more preferably 9 to 0.1, even more preferably 2.5 to 0.4. Here, the silicon compound-based binder refers to a colloid dispersion liquid, solution or emulsion of a solid or liquid silicon compound capable of being contained in an aqueous dispersion medium, specific examples of which include a colloidal silica (preferable particle size 1 to 150 nm); solutions of silicate salts such as silicate; silane and siloxane hydrolysate emulsions; a silicone resin emulsion; and emulsions of copolymers of silicone resins and other resins, such as a silicone-acrylic resin copolymer and a silicone-urethane resin copolymer.

<Method for producing titanium oxide particle dispersion liquid>

The titanium oxide particle dispersion liquid of the present invention is prepared by a production method in which a titanium oxide particle dispersion liquid and a solution or dispersion liquid of the iron, titanium and silicon components are separately produced, followed by mixing the titanium oxide particle dispersion liquid and the solution or dispersion liquid of the iron, titanium and silicon components.

As a method for producing the titanium oxide particle dispersion liquid containing the iron, titanium and silicon components, with the tin component and the visible light responsiveness-enhancing transition metal component being solid-dissolved in the titanium oxide particles, there may be specifically employed, for example, a production method having the following steps (1) to (4).

(1) A step of producing a tin component and transition metal component-containing peroxotitanic acid solution from a raw material titanium compound, tin compound, transition metal compound, basic substance, hydrogen peroxide and aqueous dispersion medium.
(2) A step of obtaining a tin component and transition metal component-containing titanium oxide particle dispersion liquid by heating the tin component and transition metal component-containing peroxotitanic acid solution produced in the step (1) at 80 to 250°C under a controlled pressure.
(3) A step of producing a solution or dispersion liquid of an iron component, titanium component and silicon component from an iron compound, titanium compound, silicon compound and aqueous dispersion medium.
(4) A step of obtaining a dispersion liquid by mixing the titanium oxide particle dispersion liquid that has been produced in the step (2) and the solution or dispersion liquid of the iron, titanium and silicon compounds that has been produced in the step (3).

The steps (1) and (2) are steps for obtaining the titanium oxide particle dispersion liquid with the tin component and the visible light responsiveness-enhancing transition metal component being solid-dissolved in the titanium oxide particles; the step (3) is a step for obtaining the solution or dispersion liquid of the iron, titanium and silicon components; and the step (4) is a step for finally obtaining the dispersion liquid containing the titanium oxide particles with the tin component and the visible light responsiveness-enhancing transition metal component being solid-dissolved therein, and with the iron, titanium and silicon components being adhered to the surfaces thereof.
As described above, as the transition metal compound used in the step (1), it is preferred that at least one kind of a molybdenum compound, tungsten compound and vanadium compound be used; each step is described in detail hereunder on such premise.

· Step (1):

In the step (1), the tin component and transition metal component-containing peroxotitanic acid solution is produced by reacting the raw material titanium compound, tin compound, transition metal compound, basic substance and hydrogen peroxide in the aqueous dispersion medium.
As a reaction method, there may be employed any of the following methods (i) to (iii).

(i) A method where the tin compound and transition metal compound are added and dissolved with respect to the raw material titanium compound and basic substance in the aqueous dispersion medium to obtain a tin component and transition metal component-containing titanium hydroxide, followed by removing impurity ions other than metal ions to be contained, and then adding hydrogen peroxide to obtain a tin component and transition metal component-containing peroxotitanic acid.
(ii) A method where the basic substance is added to the raw material titanium compound in the aqueous dispersion medium to obtain a titanium hydroxide, impurity ions other than metal ions to be contained are then removed, followed by adding the tin compound and transition metal compound, and then adding hydrogen peroxide to obtain a tin component and transition metal component-containing peroxotitanic acid.
(iii) A method where the basic substance is added to the raw material titanium compound in the aqueous dispersion medium to obtain a titanium hydroxide, impurity ions other than metal ions to be contained are then removed, hydrogen peroxide is then added to obtain a peroxotitanic acid, followed by adding the tin compound and transition metal compound to obtain a tin component and transition metal component-containing peroxotitanic acid.

Here, in the first part of the description of the method (i), "the raw material titanium compound and basic substance in the aqueous dispersion medium" may be prepared as two separate liquids of aqueous dispersion media such as "an aqueous dispersion medium with the raw material titanium compound dispersed therein" and "an aqueous dispersion medium with the basic substance dispersed therein," and each of the tin compound and transition metal compound may then be dissolved in one or both of these two liquids in accordance with the solubility of each of the tin compound and transition metal compound in these two liquids before mixing the two.
In this way, after obtaining the tin component and transition metal component-containing peroxotitanic acid, by subjecting such peroxotitanic acid to a later-described hydrothermal reaction in the step (2), there can be obtained titanium oxide particles with the various metals solid-dissolved in titanium oxide.
Here, examples of the raw material titanium compound include titanium chlorides; inorganic acid salts of titanium, such as titanium nitrate and titanium sulfate; organic acid salts of titanium, such as titanium formate, titanium citrate, titanium oxalate, titanium lactate and titanium glycolate; and titanium hydroxides precipitated by hydrolysis reactions as a result of adding alkalis to aqueous solutions of these chlorides and salts. There may be used one of them or a combination of two or more of them. Particularly, it is preferred that titanium chlorides ($TiCl_3$, $TiCl_4$) be used.

As for each of the tin compound, transition metal compound and aqueous dispersion medium, those described above are used at the compositions described above. Here, it is preferred that a concentration of a raw material titanium compound aqueous solution composed of the raw material titanium compound and aqueous dispersion medium be not higher than 60% by mass, particularly preferably not higher than 30% by mass. A lower limit of the concentration is appropriately determined; it is preferred that the lower limit be not lower than 1% by mass in general.

The basic substance is to smoothly turn the raw material titanium compound into a titanium hydroxide, examples of which include hydroxides of alkali metals or alkaline earth metals, such as sodium hydroxide and potassium hydroxide; and amine compounds such as ammonia, alkanolamine and alkylamine. Among these examples, it is particularly preferred that ammonia be used and be used in such an amount that the pH level of the raw material titanium compound aqueous solution will be 7 or higher, particularly 7 to 10. Here, the basic substance, together with the aqueous dispersion medium, may be turned into an aqueous solution having a proper concentration before use.

Hydrogen peroxide is to convert the raw material titanium compound or titanium hydroxide into a peroxotitanic acid i.e. a titanium oxide compound having a Ti-O-O-Ti bond, and is normally used in the form of a hydrogen peroxide water. It is preferred that hydrogen peroxide be added in an amount of 1.5 to 20 times the molar amount of a total substance amount of Ti, the transition metal and Sn. Further, in the reaction where hydrogen peroxide is added to turn the raw material titanium compound or titanium hydroxide into the peroxotitanic acid, it is preferred that a reaction temperature be 5 to 80°C, and that a reaction time be 30 min to 24 hours.

The tin component and transition metal component-containing peroxotitanic acid solution thus obtained may also contain an alkaline substance or acidic substance for the purpose of pH adjustment or the like. Here, examples of the alkaline substance include ammonia, sodium hydroxide, calcium hydroxide and alkylamine; examples of the acidic substance include inorganic acids such as sulfuric acid, nitric acid, hydrochloric acid, carbonic acid, phosphoric acid and hydrogen peroxide, and organic acids such as formic acid, citric acid, oxalic acid, lactic acid and glycolic acid. In this case, it is preferred that pH of the tin component and transition metal component-containing peroxotitanic acid solution obtained be 1 to 9, particularly preferably 4 to 7, in terms of safety in handling.

·Step (2):

In the step (2), the tin component and transition metal component-containing peroxotitanic acid solution obtained in the step (1) is subjected to a hydrothermal reaction under a controlled pressure and at a temperature of 80 to 250°C, preferably 100 to 250°C for 0.01 to 24 hours. An appropriate reaction temperature is 80 to 250°C in terms of reaction efficiency and reaction controllability; as a result, the tin component and transition metal component-containing peroxotitanic acid will be converted into titanium oxide particles with the tin component and the transition metal component being solid-dissolved therein. Here, the expression "under a controlled pressure" refers to a condition where if the reaction temperature is greater than the boiling point of the dispersion medium, a pressure will be applied in a proper manner such that the reaction temperature will be maintained; and even a condition where if the reaction temperature is not higher than the boiling point of the dispersion medium, atmospheric pressure will be used for control. The pressure employed here is normally about 0.12 to 4.5 MPa, preferably about 0.15 to 4.5 MPa, more preferably about 0.20 to 4.5 MPa. The reaction time is preferably 1 min to 24 hours. By this step (2), there can be obtained the dispersion liquid of the titanium oxide particles with the tin component and the transition metal component being solid-dissolved therein.

The pH of the dispersion liquid of the titanium oxide particles with the tin component and the transition metal component being solid-dissolved therein, which is obtained in the step (2), is preferably 8 to 14, more preferably 10 to 14. The dispersion liquid of the titanium oxide particles with the tin component and the transition metal component being solid-dissolved therein, which is obtained in the step (2), may also contain an alkaline substance or acidic substance for the purpose of pH adjustment or the like such that the dispersion liquid will have the abovementioned pH; the alkaline substance and acidic substance as well as a method for adjusting pH are similar to those in the case of the peroxotitanic acid solution obtained in the step (1).

It is preferred that the particle diameter ($D_{50}$ and $D_{90}$) of the titanium oxide particles obtained here fall into the ranges described above; the particle diameter can be controlled by adjusting the reaction condition(s), for example, the particle diameter can be made smaller by shortening the reaction time and a temperature rise time.

·Step (3):

In the step (3), the solution or dispersion liquid of the iron, titanium and silicon components is produced by dissolving or dispersing a raw material iron compound, raw material titanium compound and raw material silicon compound in the aqueous dispersion medium, separately from the steps (1) and (2).

As the raw material iron compound, there may be listed the abovementioned iron compounds such as elemental iron as a metal (Fe), an iron oxide ($Fe_2O_3$, $Fe_3O_4$), an iron hydroxide ($Fe(OH)_2$, $Fe(OH)_3$), an iron oxyhydroxide ($FeO(OH)$),

an iron chloride (FeClz, FeCl₃), an iron nitrate (Fe(NO)₃), an iron sulfate (FeSO₄, Fe₂(SO₄)₃), an iron halide (Br, I) other than an iron chloride, and an iron complex compound; there may be used one of them or a combination of two or more of them. Particularly, it is preferred that there be used an iron oxide ($Fe_2O_3$, $Fe_3O_4$), an iron oxyhydroxide (FeO(OH)), an iron chloride ($FeClz$, $FeCl_3$), an iron nitrate ($Fe(NO)_3$), and an iron sulfate ($FeSO_4$, $Fe_2(SO_4)_3$).

As the raw material tin compound, there may be listed the abovementioned tin compounds such as elemental titanium as a metal (Ti), a titanium hydroxide ($Ti(OH)_4$), a titanium oxyhydroxide ($TiO(OH)_2$), a titanium chloride ($TiCl_4$, $TiCl_3$, TiClz), a titanium nitrate ($Ti(NO)_4$), a titanium sulfate ($Ti(SO_4)_2$, $TiOSO_4$), a titanium halide (Br, I) other than a titanium chloride, a titanium complex compound, and a peroxotitanium compound (a titanium oxide compound having a Ti-O-O-Ti bond); there may be used one of them or a combination of two or more of them. Particularly, it is preferred that there be used a titanium hydroxide ($Ti(OH)_4$), a titanium oxyhydroxide ($TiO(OH)_2$), a titanium chloride ($TiCl_4$, $TiCl_3$, TiClz), a titanium nitrate ($Ti(NO)_4$), a titanium sulfate ($Ti(SO_4)_2$, $TiOSO_4$), and a peroxotitanium compound (a titanium oxide compound having a Ti-O-O-Ti bond).

As the raw material silicon compound, there may be listed the abovementioned silicon compounds such as elemental silicon as a metal (Si), a silicon oxide (SiO, SiOz), a silicon alkoxide ($Si(OCH_3)_4$, $Si(OC_2H_5)_4$, $Si(OCH(CH_3)_2)_4$), a silicate (sodium silicate, potassium silicate), and an active silicate obtained by removing ions such as sodium ions and potassium ions from such silicate; there may be used one of them or a combination of two or more of them. Particularly, it is preferred that there be used a silicate (sodium silicate) and an active silicate. An active silicate may for example be obtained by adding a cation-exchange resin to a sodium silicate aqueous solution prepared by dissolving sodium silicate in a pure water, and therefore removing at least part of the sodium ions; it is preferred that the cation-exchange resin be added in such a manner that an active silicate solution obtained will have a pH of 2 to 10, preferably 2 to 7.

The iron component, titanium component and silicon component-containing solution or dispersion liquid thus obtained may also contain an alkaline substance or acidic substance for the purpose of pH adjustment or the like. These alkaline substance and acidic substance may employ those similar to the ones described above, and pH adjustment here may be carried out in a similar manner as above. The iron component and silicon component-containing solution or dispersion liquid preferably has a pH of 1 to 7, more preferably 1 to 5.

In the solution or dispersion liquid of the iron, titanium and silicon components that is produced in the step (3), the concentration of the raw material iron compound is preferably 0.001 to 10% by mass, more preferably 0.01 to 5% by mass; the concentration of the raw material titanium compound is preferably 0.001 to 10% by mass, more preferably 0.01 to 5% by mass; and the concentration of the raw material silicon compound is preferably 0.001 to 10% by mass, more preferably 0.01 to 5% by mass.

·Step (4):

In the step (4), the titanium oxide particle dispersion liquid that has been obtained in the step (2) and the solution or dispersion liquid of the iron, titanium and silicon components that has been obtained in the step (3) are mixed. No particular restrictions are imposed on a mixing method; there may be used a method of performing stirring with a stirrer, or a method of performing dispersion with an ultrasonic disperser. A temperature at the time of mixing is 20 to 100°C, preferably 20 to 80°C, more preferably 20 to 40°C. A mixing time is preferably 1 min to 3 hours. In terms of a mixing ratio, mixing may be performed in such a manner that the molar ratios between $TiO_2$ and Fe, Ti and Si in the titanium oxide particle dispersion liquid shall be the above-described molar ratios.

The titanium oxide particle dispersion liquid obtained by the steps (1) to (4) may also contain an alkaline substance or acidic substance for the purpose of pH adjustment or the like; as a pH adjuster, there may be used those described above. Further, an ion-exchange treatment and a filtration washing treatment may be performed to adjust the concentration of ion components, or a solvent replacement treatment may be performed to change the solvent component. The titanium oxide particle dispersion liquid preferably has a pH of 7 to 14, more preferably 8 to 12.

The mass of the titanium oxide particles contained in the titanium oxide particle dispersion liquid can be calculated from the mass and concentration of the titanium oxide particle dispersion liquid. Here, a method for measuring the concentration of the titanium oxide particle dispersion liquid is such that part of the titanium oxide particle dispersion liquid is sampled, and the concentration is then calculated with the following formula based on the mass of a non-volatile content (titanium oxide particles) after volatilizing the solvent by performing heating at 105°C for 1 hour and the mass of the titanium oxide particle dispersion liquid sampled.

$$\text{Concentration of titanium oxide particle dispersion liquid (\%)} = [\text{mass of non-volatile content (g) / mass of titanium oxide particle dispersion liquid (g)}] \times 100$$

As described above, it is preferred that a total concentration of the iron component, titanium component, silicon component and titanium oxide particles in the titanium oxide particle dispersion liquid thus prepared be 0.01 to 20% by mass, particularly preferably 0.5 to 10% by mass, in terms of ease in producing a photocatalyst thin film having a given thickness. As for concentration adjustment, if the concentration is higher than a desired concentration, the concentration can be lowered via dilution by adding an aqueous solvent; if the concentration is lower than a desired concentration, the concentration can be raised by either volatilizing or filtering out the aqueous solvent. Here, the concentration can be calculated in the above manner.

Further, if adding the abovementioned binder enhancing a film forming capability, it is preferred that a solution of the binder (aqueous binder solution) be added to the titanium oxide particle dispersion liquid whose concentration has been adjusted in the above manner, so that the binder will be at a desired concertation after mixing.

Here, the silicon component contained in the titanium oxide particle dispersion liquid is for inhibiting the agglutination and precipitation of the titanium oxide particles, iron component and titanium component, and inhibiting deterioration in photocatalytic activity; and is added at the same time as mixing the titanium oxide particles, iron component and titanium component. Meanwhile, a binder differs from the silicon component in that it is for improving the film forming capability of the titanium oxide particle dispersion liquid, and is added after preparing the titanium oxide particle dispersion liquid before coating.

<Titanium oxide particles>

The titanium oxide particles of the present invention are characterized by having the tin component and the visible light activity-enhancing transition metal solid-dissolved therein, and the iron, titanium and silicon components adhered to the surfaces of the particles. The iron, titanium and silicon components may be adhered to at least part of or the entire surface of each titanium oxide particle.

There are no particular restrictions on a method for adhering the iron, titanium and silicon components to the surfaces of the titanium oxide particles; there may be listed, for example, a method of performing mixing in a solid state (mixing a titanium oxide particle powder with a powder made up of the iron, titanium and silicon components); a method of performing mixing in a liquid state (mixing the titanium oxide particle dispersion liquid with the solution or dispersion liquid of the iron, titanium and silicon components); and a method of mixing a solid and a liquid (mixing the solution or dispersion liquid of the iron, titanium and silicon components with the titanium oxide particle powder; or mixing the powder made up of the iron, titanium and silicon components with the titanium oxide particle dispersion liquid). Since the silicon component serves to inhibit the agglutination of the iron and titanium components, it is preferred that the iron, titanium and silicon components be previously mixed together before being mixed with the titanium oxide particles.

Further, the iron component and the titanium component may be mixed separately. Specifically, the titanium component and the silicon component may be mixed after mixing the iron and silicon components with the titanium oxide particles; or conversely, the iron component and the silicon component may be mixed after mixing the titanium and silicon components with the titanium oxide particles. As a mixing method, there may for example be used those listed above.

Among them, preferred is the method of performing mixing in a liquid state, and as is the case with the aforementioned production method of the titanium oxide particle dispersion liquid, more preferred is a method employing the steps (1) to (4).

At least part of or all the mixed iron, titanium and silicon components may be adhered to the surfaces of the titanium oxide particles. Further, it is favorable that the iron, titanium and silicon components are each directly adhered to the surfaces of the titanium oxide particles.

<Titanium oxide particle-containing photocatalyst thin film/member having photocatalyst thin film on surface>

The titanium oxide particle dispersion liquid of the present invention can be used to form photocatalyst films on the surfaces of various members. Here, no particular restrictions are imposed on the various members; examples of the materials of the members may include organic materials and inorganic materials. They may have various shapes depending on the purposes and uses thereof.

Examples of the organic materials include synthetic resin materials such as polyvinyl chloride resin (PVC), polyethylene (PE), polypropylene (PP), polycarbonate (PC), an acrylic resin, polyacetal, a fluorocarbon resin, a silicone resin, an ethylene-vinyl acetate copolymer (EVA), an acrylonitrile-butadiene rubber (NBR), polyethylene terephthalate (PET), polyethylene naphthalate (PEN), polyvinyl butyral (PVB), an ethylene-vinyl alcohol copolymer (EVOH), a polyimide resin, polyphenylene sulfide (PPS), polyetherimide (PEI), polyetheretherimide (PEEI), polyetheretherketone (PEEK), a melamine resin, a phenolic resin and an acrylonitrile-butadiene-styrene (ABS) resin; natural materials such as a natural rubber; or semisynthetic materials of the abovelisted synthetic resin materials and natural materials. It is possible that these materials have already been turned into commercial products having given shapes and structures, such as a film, sheet, fiber material, fiber product and other molded products as well as laminates.

Examples of the inorganic materials include non-metallic inorganic materials and metallic inorganic materials. Examples of the non-metallic inorganic materials include glass, ceramics and stone materials. It is possible that these materials have already been turned into commercial products having various shapes, such as tiles, glass, mirrors, walls and decorative materials. Examples of the metallic inorganic materials include a cast iron, steel, iron, iron alloy, aluminum, aluminum alloy, nickel, nickel alloy and zinc die-cast. They may be plated with any of the above metal inorganic materials or coated with any of the above organic materials, or may be used to plate the surfaces of the above organic materials or non-metallic inorganic materials.

The titanium oxide particle dispersion liquid of the present invention is especially useful for forming a photocatalyst thin film when applied to various members comprised of inorganic substances such as glass and metals, and of organic substances such as resins; the titanium oxide particle dispersion liquid of the present invention is particularly useful for forming a transparent photocatalyst thin film on various members.

As a method for forming photocatalyst thin films on the surfaces of the various members, the titanium oxide particle dispersion liquid may, for example, be applied to the surface of a member by a known application method such as spray coating and dip coating, followed by performing drying by a known drying method such as far-infrared drying, IH drying and hot-air drying. The thickness of the photocatalyst thin film may be determined variously; it is preferred that the thickness of the photocatalyst thin film normally fall into a range of 10 nm to 10 $\mu$m.

In this way, there can be formed a coating film of the titanium oxide particles. In this case, if a binder is contained in the dispersion liquid by the aforementioned amount, there can be formed a coating film containing the titanium oxide particles and the binder.

The photocatalyst thin film thus formed is transparent, and is capable of not only imparting a favorable photocatalytic action under lights in the ultraviolet region (wavelength 10 to 400 nm) as are the conventional cases, but also achieving a superior photocatalytic action even under lights in the visible region (wavelength 400 to 800 nm) of which a sufficient photocatalytic action has never been able to be achieved with a conventional photocatalyst. The various members with the photocatalyst thin films formed thereon are capable of more quickly decomposing organic substances that have adsorbed to the surfaces thereof with the aid of the photocatalytic action of titanium oxide, thereby bringing about, for example, a cleaning, deodorizing and antibacterial effects to the surfaces of the members.

WORKING EXAMPLES

The present invention is described in detail hereunder with reference to working and comparative examples. However, the present invention is not limited to the following working examples. Various measurements in the present invention were performed as follows.

(1) 50% and 90% cumulative distribution diameters of titanium oxide particles in dispersion liquid

$D_{50}$ and $D_{90}$ of the titanium oxide particles in the dispersion liquid were calculated as 50% and 90% cumulative distribution diameters on volumetric basis that are measured by a dynamic light scattering method using a laser light, by means of a particle size distribution measurement device (ELSZ-2000ZS by Otsuka Electronics Co., Ltd.).

(2) Acetaldehyde gas decomposition capability test of photocatalyst thin film

The activity of a photocatalyst thin film produced by applying the dispersion liquid and then drying the same was evaluated through a decomposition reaction of an acetaldehyde gas. The evaluation was performed by a batch-wise gas decomposition capability evaluation method.

Using a #7 wire bar coater, each titanium oxide particle dispersion liquid prepared in the working or comparative examples was applied to and spread on the entire surface of an A4-sized PET film (210 mm $\times$ 297 mm) in a way such that a dry mass of the titanium oxide particles would be about 20 mg. A sample thus prepared was then dried in an oven of 80°C for 1 hour to obtain an evaluation sample for acetaldehyde gas decomposition capability evaluation.

Using such evaluation sample, the photocatalytic activity of the titanium oxide particles was evaluated via a decomposition reaction of acetaldehyde gas. The evaluation was performed by a batch-wise gas decomposition capability evaluation method.

Specifically, the evaluation sample was at first placed into a 5L stainless cell equipped with a quartz glass window. This cell was then filled with an acetaldehyde gas having an initial concentration with a humidity thereof being controlled to 50%, followed by performing light irradiation with a light source provided at an upper portion of the cell. As a result of having the acetaldehyde gas decomposed by the photocatalytic action of titanium oxide, the acetaldehyde gas concentration in the cell will decrease. There, by measuring a change in the concentration, the intensity of photocatalytic activity can be confirmed. The acetaldehyde gas concentration was measured by a photoacoustic multi-gas monitor (product name "INNOVA1412" by LumaSense Technologies), and the photocatalytic activity was evaluated by measuring a time it took for the acetaldehyde gas concentration to reach 1 ppm or lower after the light irradiation was initiated. The shorter the time measured is, the higher the photocatalytic activity is; the longer the time measured is, the lower the photocatalytic activity is.

In a photocatalytic activity evaluation under visible light irradiation, an LED (product model number "TH-211×200SW" by CCS Inc., spectral distribution: 400 to 800 nm) was used as a light source, and visible light irradiation was carried out at an illuminance of 10,000 Lx. At that time, the initial concentration of the acetaldehyde in the cell was set to 5 ppm.

Further, in a photocatalytic activity evaluation under ultraviolet irradiation, a UV fluorescent lamp (product model number "FL10 BLB" by Toshiba Lighting & Technology Corporation) was used as a light source, and ultraviolet irradiation (352 nm) was carried out at an irradiance of 0.2 mW/cm$^2$. At that time, the initial concentration of the acetaldehyde in the cell was set to 20 ppm.

(3) Identification of crystal phase of titanium oxide particles

The crystal phase of the titanium oxide particles was identified in a way where the dispersion liquid of the titanium oxide particles obtained was dried at 105°C for three hours to obtain a titanium oxide particle powder, followed by collecting the titanium oxide particle powder so as to subject the same to powder X-ray diffraction analysis, using a diffraction device (product name "Benchtop X-ray diffractometer D2 PHASER" by BRUKER AXS Co., Ltd.).

(4) Preparation of titanium oxide particle dispersion liquid

[Preparation example 1-1]

<Preparation of dispersion liquid of titanium oxide particles with tin and molybdenum solid-dissolved therein>

Tin (IV) chloride was added to and dissolved in a 36% by mass titanium (IV) chloride aqueous solution so that $TiO_2$/Sn (molar ratio) in the titanium oxide particle dispersion liquid obtained in the preparation example 1-1 would be 20, followed by diluting the solution thus prepared 10 times with a pure water, and then neutralizing and hydrolyzing the same by gradually adding a 10% by mass ammonia water, thereby obtaining a precipitate of a tin-containing titanium hydroxide. pH at that time was 8. The precipitate thus obtained was then deionized by repeating the addition of pure water and decantation. Sodium molybdate (VI) was then added to the deionized precipitate of the tin-containing titanium hydroxide so that $TiO_z$/Mo (molar ratio) in the titanium oxide particle dispersion liquid obtained in the preparation example 1-1 would be 400. A 35% by mass hydrogen peroxide water was then added so that $H_2O_2$/(Ti+Sn+Mo) (molar ratio) would be 10, followed by performing stirring at 60°C for two hours so as to sufficiently react the solution, thereby obtaining an orange transparent tin and molybdenum-containing peroxotitanic acid solution (1a).

Next, 400 mL of the tin and molybdenum-containing peroxotitanic acid solution (1a) was put into a 500 mL autoclave so as to be subjected to a hydrothermal treatment at 150°C for 90 min, followed by adding a pure water to adjust the concentration thereof, thereby obtaining a dispersion liquid of titanium oxide particles (1A) with tin and molybdenum solid-dissolved therein (titanium oxide concentration 1.2% by mass). As a result of performing powder X-ray diffraction analysis on the titanium oxide particles (1A), there were only observed peaks of a rutile-type titanium oxide; it was confirmed that tin and molybdenum were solid-dissolved in titanium oxide.

[Preparation example 1-2]

<Preparation of dispersion liquid of titanium oxide particles with tin and tungsten solid-dissolved therein>

Tin (IV) chloride was added to and dissolved in a 36% by mass titanium (IV) chloride aqueous solution so that $TiO_2$/Sn (molar ratio) in the titanium oxide particle dispersion liquid obtained in the preparation example 1-2 would be 10, followed by diluting the solution thus prepared 10 times with a pure water, and then neutralizing and hydrolyzing the same by gradually adding a 10% by mass ammonia water, thereby obtaining a precipitate of a tin-containing titanium hydroxide. pH at that time was 8. The precipitate thus obtained was then deionized by repeating the addition of pure water and decantation. Sodium tungstate (VI) was then added to the deionized precipitate of the tin-containing titanium hydroxide so that $TiO_2$/W (molar ratio) in the titanium oxide particle dispersion liquid obtained in the preparation example 1-2 would be 100. A 35% by mass hydrogen peroxide water was then added so that $H_2O_2$/(Ti+Sn+W) (molar ratio) would be 10, followed by performing stirring at 60°C for two hours so as to sufficiently react the solution, thereby obtaining an orange transparent tin and tungsten-containing peroxotitanic acid solution (1b).

Next, 400 mL of the tin and tungsten-containing peroxotitanic acid solution (1b) was put into a 500 mL autoclave so as to be subjected to a hydrothermal treatment at 160°C for 60 min, followed by adding a pure water to adjust the concentration thereof, thereby obtaining a dispersion liquid of titanium oxide particles (1B) with tin and tungsten solid-dissolved therein (titanium oxide concentration 1.2% by mass). As a result of performing powder X-ray diffraction analysis on the titanium oxide particles (1B), there were only observed peaks of a rutile-type titanium oxide; it was confirmed that tin and tungsten were solid-dissolved in titanium oxide.

[Preparation example 1-3]

<Preparation of dispersion liquid of titanium oxide particles with tin and vanadium solid-dissolved therein>

Tin (IV) chloride was added to and dissolved in a 36% by mass titanium (IV) chloride aqueous solution so that $TiO_2/Sn$ (molar ratio) in the titanium oxide particle dispersion liquid obtained in the preparation example 1-3 would be 33, followed by diluting the solution thus prepared 10 times with a pure water, and then neutralizing and hydrolyzing the same by gradually adding a 10% by mass ammonia water, thereby obtaining a precipitate of a tin-containing titanium hydroxide. pH at that time was 8. The precipitate thus obtained was then deionized by repeating the addition of pure water and decantation. Sodium vanadate (V) was then added to the deionized precipitate of the tin-containing titanium hydroxide so that $TiO_2/V$ (molar ratio) in the titanium oxide particle dispersion liquid obtained in the preparation example 1-3 would be 2,000. A 35% by mass hydrogen peroxide water was then added so that $H_2O_2/(Ti+Sn+V)$ (molar ratio) would be 10, followed by performing stirring at 50°C for three hours so as to sufficiently react the solution, thereby obtaining an orange transparent tin and vanadium-containing peroxotitanic acid solution (1c).

Next, 400 mL of the tin and vanadium-containing peroxotitanic acid solution (1c) was put into a 500 mL autoclave so as to be subjected to a hydrothermal treatment at 140°C for 120 min, followed by adding a pure water to adjust the concentration thereof, thereby obtaining a dispersion liquid of titanium oxide particles (1C) with tin and vanadium solid-dissolved therein (titanium oxide concentration 1.2% by mass). As a result of performing powder X-ray diffraction analysis on the titanium oxide particles (1C), there were observed peaks of an anatase-type titanium oxide and a rutile-type titanium oxide; it was confirmed that tin and vanadium were solid-dissolved in titanium oxide.

[Preparation example 1-4]

<Preparation of dispersion liquid of titanium oxide particles with tin and molybdenum solid-dissolved therein>

Tin (IV) chloride was added to and dissolved in a 36% by mass titanium (IV) chloride aqueous solution so that $TiO_2/Sn$ (molar ratio) in the titanium oxide particle dispersion liquid obtained in the preparation example 1-4 would be 20, followed by diluting the solution thus prepared 10 times with a pure water, and then neutralizing and hydrolyzing the same by gradually adding a 10% by mass ammonia water, thereby obtaining a precipitate of a tin-containing titanium hydroxide. pH at that time was 8. The precipitate thus obtained was then deionized by repeating the addition of pure water and decantation. Sodium molybdate (VI) was then added to the deionized precipitate of the tin-containing titanium hydroxide so that $TiO_2/Mo$ (molar ratio) in the titanium oxide particle dispersion liquid obtained in the preparation example 1-4 would be 100. A 35% by mass hydrogen peroxide water was then added so that $H_2O_2/(Ti+Sn+Mo)$ (molar ratio) would be 12, followed by performing stirring at 60°C for two hours so as to sufficiently react the solution, thereby obtaining an orange transparent tin and molybdenum-containing peroxotitanic acid solution (1d).

Next, 400 mL of the tin and molybdenum-containing peroxotitanic acid solution (1d) was put into a 500 mL autoclave so as to be subjected to a hydrothermal treatment at 120°C for 180 min, followed by adding a pure water to adjust the concentration thereof, thereby obtaining a dispersion liquid of titanium oxide particles (1D) with tin and molybdenum solid-dissolved therein (titanium oxide concentration 1.2% by mass). As a result of performing powder X-ray diffraction analysis on the titanium oxide particles (1D), there were only observed peaks of a rutile-type titanium oxide; it was confirmed that tin and molybdenum were solid-dissolved in titanium oxide.

[Preparation example 1-5]

<Preparation of dispersion liquid of titanium oxide particles with tin, tungsten and vanadium solid-dissolved therein>

Tin (IV) chloride was added to and dissolved in a 36% by mass titanium (IV) chloride aqueous solution so that $TiO_2/Sn$ (molar ratio) in the titanium oxide particle dispersion liquid obtained in the preparation example 1-5 would be 50, followed by diluting the solution thus prepared 10 times with a pure water, and then neutralizing and hydrolyzing the same by gradually adding a 10% by mass ammonia water, thereby obtaining a precipitate of a tin-containing titanium hydroxide. pH at that time was 8. The precipitate thus obtained was then deionized by repeating the addition of pure water and decantation. Sodium tungstate (VI) was then added to the deionized precipitate of the tin-containing titanium hydroxide so that $TiO_2/W$ (molar ratio) in the titanium oxide particle dispersion liquid obtained in the preparation example 1-5 would be 33, and sodium vanadate (V) was added to the deionized precipitate so that TiOz/V (molar ratio) in such titanium oxide particle dispersion liquid would be 5,000. A 35% by mass hydrogen peroxide water was then added so that $H_2O_2/(Ti+Sn+W+V)$ (molar ratio) would be 10, followed by performing stirring at 60°C for two hours so as to sufficiently react the solution, thereby obtaining an orange transparent tin and tungsten-containing peroxotitanic acid solution (1e).

Next, 400 mL of the tin, tungsten and vanadium-containing peroxotitanic acid solution (1e) was put into a 500 mL autoclave so as to be subjected to a hydrothermal treatment at 140°C for 120 min, followed by adding a pure water to adjust the concentration thereof, thereby obtaining a dispersion liquid of titanium oxide particles (1E) with tin, tungsten and vanadium solid-dissolved therein (titanium oxide concentration 1.2% by mass). As a result of performing powder X-ray diffraction analysis on the titanium oxide particles (1E), there were observed peaks of an anatase-type titanium oxide and a rutile-type titanium oxide; it was confirmed that tin, tungsten and vanadium were solid-dissolved in titanium oxide.

[Preparation example 1-6]

<Preparation of dispersion liquid of titanium oxide particles with tin solid-dissolved therein>

A dispersion liquid of titanium oxide particles (1F) with tin solid-dissolved therein (titanium oxide concentration 1.2% by mass) was obtained in a similar manner as the preparation example 1-1, except that sodium molybdate (VI) was not added. As a result of performing powder X-ray diffraction analysis on the titanium oxide particles (1F), there were only observed peaks of a rutile-type titanium oxide; it was confirmed that tin was solid-dissolved in titanium oxide.

[Preparation example 1-7]

<Preparation of dispersion liquid of titanium oxide particles with molybdenum solid-dissolved therein>

A dispersion liquid of titanium oxide particles (1G) with molybdenum solid-dissolved therein (titanium oxide concentration 1.2% by mass) was obtained in a similar manner as the preparation example 1-1, except that tin (IV) chloride was not added. As a result of performing powder X-ray diffraction analysis on the titanium oxide particles (1G), there were only observed peaks of an anatase-type titanium oxide; it was confirmed that molybdenum was solid-dissolved in titanium oxide.

[Preparation example 1-8]

<Preparation of dispersion liquid of titanium oxide particles with tungsten solid-dissolved therein>

A dispersion liquid of titanium oxide particles (1H) with tungsten solid-dissolved therein (titanium oxide concentration 1.2% by mass) was obtained in a similar manner as the preparation example 1-2, except that tin (IV) chloride was not added. As a result of performing powder X-ray diffraction analysis on the titanium oxide particles (1H), there were only observed peaks of an anatase-type titanium oxide; it was confirmed that tungsten was solid-dissolved in titanium oxide.

[Preparation example 1-9]

<Preparation of dispersion liquid of titanium oxide particles with vanadium solid-dissolved therein>

A dispersion liquid of titanium oxide particles (1I) with vanadium solid-dissolved therein (titanium oxide concentration 1.2% by mass) was obtained in a similar manner as the preparation example 1-3, except that tin (IV) chloride was not added. As a result of performing powder X-ray diffraction analysis on the titanium oxide particles (1I), there were only observed peaks of an anatase-type titanium oxide; it was confirmed that vanadium was solid-dissolved in titanium oxide.

Shown collectively in Table 1 are the molar ratios, hydrothermal treatment conditions, dispersion particle diameters ($D_{50}$, $D_{90}$) and pH of the titanium oxide particle dispersion liquid after the hydrothermal treatment with regard to the titanium oxide particles prepared in each preparation example. The dispersion particle diameters were measured by a dynamic light scattering method using a laser light (ELSZ-2000ZS by Otsuka Electronics Co., Ltd.).

[Table 1]

| Preparation example | Titanium oxide particle dispersion liquid | Molar ratio | | | | Hydrothermal treatment | | Particle diameter | | pH |
|---|---|---|---|---|---|---|---|---|---|---|
| | | $TiO_2/Sn$ | $TiO_2/Mo$ | $TiO_2/W$ | $TiO_2/V$ | Temperaure °C | Time min | $D_{50}$ nm | $D_{90}$ nm | |
| 1-1 | 1 A | 20 | 400 | - | - | 150 | 90 | 8 | 14 | 10.5 |
| 1 - 2 | 1 B | 10 | - | 100 | - | 160 | 60 | 7 | 13 | 10.2 |
| 1 - 3 | 1 C | 33 | - | - | 2000 | 140 | 120 | 13 | 28 | 10.6 |
| 1-4 | 1D | 20 | 100 | - | - | 120 | 180 | 11 | 24 | 10.7 |
| 1 - 5 | 1 E | 50 | - | 33 | 5000 | 140 | 120 | 15 | 29 | 10.6 |
| 1 - 6 | 1 F | 20 | - | - | - | 150 | 90 | 9 | 19 | 10.5 |
| 1 - 7 | 1 G | - | 400 | - | - | 150 | 90 | 18 | 26 | 10.5 |
| 1 - 8 | 1 H | - | - | 100 | - | 160 | 60 | 20 | 39 | 10.2 |
| 1 - 9 | 1 I | - | - | | 2000 | 140 | 120 | 19 | 37 | 10.6 |

(5) Preparation of solution or dispersion liquid of iron, titanium and silicon components

[Preparation example 2-1]

<Preparation of aqueous solution of iron sulfate, titanium chloride and active silicate>

A strong acid cation-exchange resin (AMBERJET 1024H by ORGANO CORPORATION) was added to a silicate soda aqueous solution obtained by dissolving 0.34 g of JIS No.3 silicate soda (29.1% by mass in terms of $SiO_2$) into 100 g of a pure water. After performing stirring, the ion-exchange resin was removed by filtration to obtain an active silicate aqueous solution. By adding 0.13 g of iron (III) sulfate and 0.12 g of titanium (IV) chloride to this active silicate aqueous solution, there was obtained a pH 1.8 aqueous solution (2A) of iron sulfate, titanium chloride and active silicate.

[Preparation example 2-2]

<Preparation of aqueous solution of iron sulfate, titanium chloride and active silicate>

A strong acid cation-exchange resin (AMBERJET 1024H by ORGANO CORPORATION) was added to a silicate soda aqueous solution obtained by dissolving 3.43 g of JIS No.3 silicate soda (29.1% in terms of $SiO_2$) into 100 g of a pure water. After performing stirring, the ion-exchange resin was removed by filtration to obtain an active silicate aqueous solution. By adding 0.38 g of iron (III) sulfate and 0.02 g of titanium (IV) chloride to this active silicate aqueous solution, there was obtained a pH 2.2 aqueous solution (2B) of iron sulfate, titanium chloride and active silicate.

[Preparation example 2-3]

<Preparation of aqueous solution of iron sulfate, titanium chloride and active silicate>

A strong acid cation-exchange resin (AMBERJET 1024H by ORGANO CORPORATION) was added to a silicate soda aqueous solution obtained by dissolving 0.17 g of JIS No.3 silicate soda (29.1% in terms of $SiO_2$) into 100 g of a pure water. After performing stirring, the ion-exchange resin was removed by filtration to obtain an active silicate aqueous solution. By adding 0.06 g of iron (III) sulfate and 0.06 g of titanium (IV) chloride to this active silicate aqueous solution, there was obtained a pH 2.2 aqueous solution (2C) of iron sulfate, titanium chloride and active silicate.

[Preparation example 2-4]

<Preparation of aqueous solution of iron sulfate and active silicate>

A pH 2.6 aqueous solution (2D) of iron sulfate and active silicate was obtained in a similar manner as the preparation example 2-3, except that titanium (IV) chloride was not added.

[Preparation example 2-5]

<Preparation of aqueous solution of titanium chloride and active silicate>

A pH 2.2 aqueous solution (2E) of titanium chloride and active silicate was obtained in a similar manner as the preparation example 2-3, except that iron (III) sulfate was not added.

[Preparation example 2-6]

<Preparation of iron sulfate aqueous solution>

A pH 2.5 iron sulfate aqueous solution (2F) was obtained in a similar manner as the preparation example 2-2, except that the JIS No.3 silicate soda and titanium (IV) chloride were not added.

[Preparation example 2-7]

<Preparation of titanium chloride aqueous solution>

A pH 1.8 titanium chloride aqueous solution (2G) was obtained in a similar manner as the preparation example 2-1, except that the JIS No.3 silicate soda and iron (III) sulfate were not added.

[Preparation example 2-8]

<Preparation of active silicate aqueous solution>

A pH 4.8 active silicate aqueous solution (2H) was obtained in a similar manner as the preparation example 2-1, except that iron sulfate and titanium chloride were not added.

(6) Preparation of titanium oxide particle dispersion liquid

[Working example 1]

A stirrer was used to mix the aqueous solution (2A) of iron sulfate, titanium chloride and active silicate with the dispersion liquid of the titanium oxide particles (1A) at 25°C for 10 min so that $TiO_2$/Fe, $TiO_z$/Ti and $TiO_2$/Si would respectively be 400, 200 and 75, followed by adjusting the solid content concentration to 1% by mass with a pure water, thus obtaining a titanium oxide particle dispersion liquid (E-1).

[Working example 2]

A stirrer was used to mix the aqueous solution (2B) of iron sulfate, titanium chloride and active silicate with the dispersion liquid of the titanium oxide particles (1B) at 25°C for 10 min so that $TiO_2$/Fe, $TiO_z$/Ti and $TiO_2$/Si would respectively be 133, 1,000 and 8, followed by adjusting the solid content concentration to 1% by mass with a pure water, thus obtaining a titanium oxide particle dispersion liquid (E-2).

[Working example 3]

A stirrer was used to mix the aqueous solution (2C) of iron sulfate, titanium chloride and active silicate with the dispersion liquid of the titanium oxide particles (1C) at 25°C for 10 min so that $TiO_2$/Fe, $TiO_z$/Ti and $TiO_2$/Si would respectively be 800, 400 and 150, followed by adjusting the solid content concentration to 1% by mass with a pure water, thus obtaining a titanium oxide particle dispersion liquid (E-3).

[Working example 4]

A stirrer was used to mix the aqueous solution (2A) of iron sulfate, titanium chloride and active silicate with the dispersion liquid of the titanium oxide particles (1D) at 25°C for 10 min so that $TiO_2$/Fe, $TiO_z$/Ti and $TiO_2$/Si would respectively be 400, 200 and 75, followed by adjusting the solid content concentration to 1% by mass with a pure water, thus obtaining a titanium oxide particle dispersion liquid (E-4).

[Working example 5]

A stirrer was used to mix the aqueous solution (2A) of iron sulfate, titanium chloride and active silicate with the dispersion liquid of the titanium oxide particles (1E) at 25°C for 10 min so that $TiO_2$/Fe, $TiO_z$/Ti and $TiO_2$/Si would respectively be 400, 200 and 75, followed by adjusting the solid content concentration to 1% by mass with a pure water, thus obtaining a titanium oxide particle dispersion liquid (E-5).

[Working example 6]

A silicon compound-based (silica-based) binder (colloidal silica, product name "SNOWTEX 20" by Nissan Chemical Corporation) was added to the titanium oxide particle dispersion liquid (E-4) so that $TiO_2$/$SiO_2$ (mass ratio) would be 1.5, and was mixed with this dispersion liquid by a stirrer at 25°C for 10 min, thus obtaining a binder-containing titanium oxide particle dispersion liquid (E-6).

[Comparative example 1]

A stirrer was used to mix the aqueous solution (2A) of iron sulfate, titanium chloride and active silicate with the dispersion liquid of the titanium oxide particles (1F) at 25°C for 10 min so that $TiO_2$/Fe, $TiO_z$/Ti and $TiO_2$/Si would respectively be 400, 200 and 75, followed by adjusting the solid content concentration to 1% by mass with a pure water, thus obtaining a titanium oxide particle dispersion liquid (C-1).

[Comparative example 2]

A stirrer was used to mix the aqueous solution (2A) of iron sulfate, titanium chloride and active silicate with the dispersion liquid of the titanium oxide particles (1G) at 25°C for 10 min so that $TiO_2/Fe$, $TiOz/Ti$ and $TiO_2/Si$ would respectively be 400, 200 and 75, followed by adjusting the solid content concentration to 1% by mass with a pure water, thus obtaining a titanium oxide particle dispersion liquid (C-2).

[Comparative example 3]

A stirrer was used to mix the aqueous solution (2A) of iron sulfate, titanium chloride and active silicate with the dispersion liquid of the titanium oxide particles (1H) at 25°C for 10 min so that $TiO_2/Fe$, $TiOz/Ti$ and $TiO_2/Si$ would respectively be 400, 200 and 75, followed by adjusting the solid content concentration to 1% by mass with a pure water, thus obtaining a titanium oxide particle dispersion liquid (C-3).

[Comparative example 4]

A stirrer was used to mix the aqueous solution (2A) of iron sulfate, titanium chloride and active silicate with the dispersion liquid of the titanium oxide particles (1I) at 25°C for 10 min so that $TiO_2/Fe$, $TiOz/Ti$ and $TiO_2/Si$ would respectively be 400, 200 and 75, followed by adjusting the solid content concentration to 1% by mass with a pure water, thus obtaining a titanium oxide particle dispersion liquid (C-4).

[Comparative example 5]

A stirrer was used to mix the aqueous solution (2D) of iron sulfate and active silicate with the dispersion liquid of the titanium oxide particles (1D) at 25°C for 10 min so that $TiO_2/Fe$ and $TiOz/Si$ would respectively be 800 and 150, followed by adjusting the solid content concentration to 1% by mass with a pure water, thus obtaining a titanium oxide particle dispersion liquid (C-5).

[Comparative example 6]

A stirrer was used to mix the aqueous solution (2E) of titanium chloride and active silicate with the dispersion liquid of the titanium oxide particles (1D) at 25°C for 10 min so that $TiOz/Ti$ and $TiOz/Si$ would respectively be 400 and 150, followed by adjusting the solid content concentration to 1% by mass with a pure water, thus obtaining a titanium oxide particle dispersion liquid (C-6).

[Comparative example 7]

A stirrer was used to mix the iron sulfate aqueous solution (2F) with the dispersion liquid of the titanium oxide particles (1D) at 25°C for 10 min so that $TiO_2/Fe$ would be 133, followed by adjusting the solid content concentration to 1% by mass with a pure water, thus obtaining a titanium oxide particle dispersion liquid (C-7).
The addition of iron sulfate caused the titanium oxide particles to agglutinate, and part of them to precipitate, which led to a white and turbid appearance of the dispersion liquid. Further, as a result of filtrating the titanium oxide particle dispersion liquid (C-7) with a PP filter having a pore diameter of 1 $\mu$m, a brown iron component was found present on the filter, which indicated that the iron component had agglutinated as well.

[Comparative example 8]

A stirrer was used to mix the titanium chloride aqueous solution (2G) with the dispersion liquid of the titanium oxide particles (1D) at 25°C for 10 min so that $TiOz/Ti$ would be 200, followed by adjusting the solid content concentration to 1% by mass with a pure water, thus obtaining a titanium oxide particle dispersion liquid (C-8).
The addition of titanium chloride caused the titanium oxide particles to agglutinate, and part of them to precipitate, which led to a white and turbid appearance of the dispersion liquid. Further, as a result of filtrating the titanium oxide particle dispersion liquid (C-8) with a PP filter having a pore diameter of 1 $\mu$m, a hydrous white component was found present on the filter, which indicated that the titanium component had also agglutinated in addition to titanium oxide.

[Comparative example 9]

A stirrer was used to mix the active silicate aqueous solution (2H) with the dispersion liquid of the titanium oxide particles (1D) at 25°C for 10 min so that TiOz/Si would be 75, followed by adjusting the solid content concentration to 1% by mass with a pure water, thus obtaining a titanium oxide particle dispersion liquid (C-9).

[Comparative example 10]

The solid content concentration of the dispersion liquid of the titanium oxide particles (1D) was adjusted to 1% by mass with a pure water, thus obtaining a titanium oxide particle dispersion liquid (C-10).

[Comparative example 11]

A silicon compound-based (silica-based) binder (colloidal silica, product name "SNOWTEX 20" by Nissan Chemical Corporation) was added to the titanium oxide particle dispersion liquid (C-5) so that $TiO_2/SiO_2$ (mass ratio) would be 1.5, and was mixed with this dispersion liquid by a stirrer at 25°C for 10 min, thus obtaining a binder-containing titanium oxide particle dispersion liquid (C-11).

[Comparative example 12]

A silicon compound-based (silica-based) binder (colloidal silica, product name "SNOWTEX 20" by Nissan Chemical Corporation) was added to the titanium oxide particle dispersion liquid (C-6) so that $TiO_2/SiO_2$ (mass ratio) would be 1.5, and was mixed with this dispersion liquid by a stirrer at 25°C for 10 min, thus obtaining a binder-containing titanium oxide particle dispersion liquid (C-12).

(7) Production of sample member having photocatalyst thin film

Using the #7 wire bar coater, each titanium oxide particle dispersion liquid prepared in the working or comparative examples was applied to the A4-sized PET film so as to be able to form thereon a photocatalyst thin film (having a thickness of about 80 nm) containing 20 mg of titanium oxide particles. The PET film coated was then dried in an oven of 80°C for 1 hour to obtain a sample member for acetaldehyde gas decomposition capability evaluation.

[Photocatalytic capability test under visible light irradiation]

An acetaldehyde decomposition test was performed on the sample members having the photocatalyst thin films of the working and comparative examples under an irradiation of a visible light by LED. Evaluation was conducted based on the time it took for the acetaldehyde concentration to be reduced from 5 ppm which was an initial concentration to 1 ppm.

Here, cases where the acetaldehyde concentration failed to be reduced to 1 ppm in 24 hours were marked with "-" in a column titled "Time taken to be decomposed to 1 ppm" in Tables 2 and 3, and the corresponding concentrations after 24 hours are shown in a column titled "Concentration after 24 h" in these tables.

Shown collectively in Table 2 are the types of the titanium oxide particles and added metal components in the working examples 1 to 5 and comparative examples 1 to 10; the molar ratios of the metals in the added metal components to titanium oxide (TiOz); the dispersion particle diameters ($D_{50}$, $D_{90}$); pHs; and the results of the acetaldehyde gas decomposition test performed under visible light irradiation. The dispersion particle diameters were measured by a dynamic light scattering method using a laser light (ELSZ-2000ZS by Otsuka Electronics Co., Ltd.).

[Table 2]

| | (Evaluation sample) Titanium oxide particle dispersion liquid | Titanium oxide particle dispersion liquid | Molar ratio of component solid-dissolved in titanium oxide to $TiO_2$ | | | | Aqueous solution or dispersionliquid of added component | Molar ratio to $TiO_2$ in titanium oxide particle dispersion liquid (except for component solid-dissolved in titanium oxide) | | | Evaluation results | | | | |
|---|---|---|---|---|---|---|---|---|---|---|---|---|---|---|---|
| | | | $TiO_2/Sn$ | $TiO_2/Mo$ | $TiO_2/W$ | $TiO_2/V$ | | $TiO_2/Fe$ | $TiO_2/Ti$ | $TiO_2/Si$ | $D_{50}$ (nm) | $D_{90}$ (nm) | pH | Time taken to 1 ppm (hr) | Concent-ration after 24 hours (ppm) |
| Working example 1 | E-1 | 1A | 20 | 400 | - | - | 2A | 400 | 200 | 75 | 10 | 22 | 10.3 | 4.9 | - |
| Working example 2 | E-2 | 1B | 10 | - | 100 | - | 2B | 133 | 1000 | 8 | 12 | 28 | 10.1 | 6.5 | - |
| Working example 3 | E-3 | 1C | 33 | - | - | 2000 | 2C | 800 | 400 | 150 | 14 | 30 | 10.4 | 9.2 | - |
| Working example 4 | E-4 | 1D | 20 | 100 | - | - | 2A | 400 | 200 | 75 | 13 | 29 | 10.5 | 5.4 | - |
| Working example 5 | E-5 | 1E | 50 | - | 33 | 5000 | 2A | 400 | 200 | 75 | 17 | 38 | 10.4 | 8.7 | - |
| Comparative example 1 | C-1 | 1F | 20 | - | - | - | 2A | 400 | 200 | 75 | 11 | 25 | 10.3 | - | 3.8 |
| Comparative example 2 | C-2 | 1G | - | 400 | - | - | 2A | 400 | 200 | 75 | 21 | 45 | 10.3 | - | 4.8 |
| Comparative example 3 | C-3 | 1H | - | - | 100 | - | 2A | 400 | 200 | 75 | 23 | 50 | 10.1 | - | 4.5 |
| Comparative example 4 | C-4 | 1I | - | - | - | 2000 | 2A | 400 | 200 | 75 | 22 | 48 | 10.3 | - | 4.8 |
| Comparative example 5 | C-5 | 1D | 20 | 100 | - | - | 2D | 800 | - | 150 | 12 | 28 | 10.6 | 18.5 | - |
| Comparative example 6 | C-6 | 1D | 20 | 100 | - | - | 2E | - | 400 | 150 | 14 | 29 | 10.4 | - | 4.6 |

| | (Evaluation sample) Titanium oxide particle dispersion liquid | Titanium oxide particle dispersion liquid | Molar ratio of component solid-dissolved in titanium oxide to $TiO_2$ | | | | Aqueous solution or dispersionliquid of added component | Molar ratio to $TiO_2$ in titanium oxide particle dispersion liquid (except for component solid-dissolved in titanium oxide) | | | Evaluation results | | | | |
|---|---|---|---|---|---|---|---|---|---|---|---|---|---|---|---|
| | | | $TiO_2/Sn$ | $TiO_2/Mo$ | $TiO_2/W$ | $TiO_2/V$ | | $TiO_2/Fe$ | $TiO_2/Ti$ | $TiO_2/Si$ | $D_{50}$ (nm) | $D_{90}$ (nm) | pH | Time taken to 1 ppm (hr) | Concent-ration after 24 hours (ppm) |
| Comparative example 7 | C-7 | 1D | 20 | 100 | - | - | 2 F | 133 | - | - | - | - | 10.6 | - | - |
| Comparative example 8 | C-8 | 1 D | 20 | 100 | - | - | 2G | - | 200 | - | - | - | 10.5 | - | - |
| Comparative example 9 | C-9 | 1 D | 20 | 100 | - | - | 2H | - | - | 75 | 12 | 28 | 10.7 | - | 4.7 |
| Comparative example 10 | C-10 | 1 D | 20 | 100 | - | - | - | - | - | - | 11 | 24 | 10.8 | - | 4.6 |

As can seen from the results of the working examples 1 to 5 and comparative examples 1 to 4, by employing the titanium oxide particles with both the tin component and the visible light activity-enhancing transition metal component solid-dissolved therein, a higher photocatalytic activity under visible light irradiation was able to be achieved as compared to when employing the titanium oxide with only one or none of the tin component and the visible light activity-enhancing transition metal component solid-dissolved therein.

As can be seen from the results of the working example 4 and comparative examples 5, 6, 9 and 10, the usage of the photocatalyst titanium oxide with the iron, titanium and silicon components (2A) adhered to the surfaces of the titanium oxide particles (1D) led to a higher photocatalytic activity under visible light irradiation as compared to when employing the photocatalyst titanium oxide with the iron and silicon components (2D) adhered thereto, the photocatalyst titanium oxide with the titanium and silicon components (2E) adhered thereto, the photocatalyst titanium oxide with the silicon component (2H) adhered thereto, and the photocatalyst titanium oxide with none of these components adhered thereto.

As can be seen from the results of the working example 4 and comparative examples 7 and 8, when adding the iron and titanium components to the titanium oxide particles (1D), by also adding the silicon component, the titanium oxide particles, iron component and titanium component were able to be inhibited from agglutinating and precipitating.

As such, a superior photocatalytic capability was observed with the titanium oxide particles of the present invention with the iron, titanium and silicon components being adhered to the surfaces thereof, and with the tin component and the visible light activity-enhancing transition metal component being solid-dissolved therein.

[Photocatalytic capability test under UV irradiation]

An acetaldehyde decomposition test was performed on the sample members having the photocatalyst thin films of the working example 6 and comparative examples 11 and 12 under an irradiation by a UV fluorescent lamp. Evaluation was conducted based on the time it took for the acetaldehyde concentration to be reduced from 20 ppm which was an initial concentration to 1 ppm.

Shown collectively in Table 3 are the types of the titanium oxide particles and added metals; the molar ratios of the metals in the added metal components to titanium oxide (TiOz); the dispersion particle diameters ($D_{50}$, $D_{90}$); pHs; and the results of the acetaldehyde gas decomposition test. The dispersion particle diameters were measured by a dynamic light scattering method using a laser light (ELSZ-2000ZS by Otsuka Electronics Co., Ltd.).

[Table 3]

| | (Evaluation sample) Titanium oxide particle dispersion liquid | Titanium oxide particle dispersion liquid | Molar ratio of component solid-dissolved in titanium oxide to $TiO_2$ | | | | Aqueous solution or dispersion liquid of added component | Molar ratio to $TiO_2$ in titanium oxide particle dispersion liquid (except for component solid-dissolved in titanium oxide) | | | Evaluation results | | | | |
| | | | $TiO_2$/Sn | $TiO_2$/Mo | $TiO_2$/W | $TiO_2$/V | | $TiO_2$/Fe | $TiO_2$/Ti | $TiO_2$/Si | $D_{50}$ (nm) | $D_{90}$ (nm) | pH | Time taken to 1 ppm (hr) | Concentration after 24 hours (ppm) |
|---|---|---|---|---|---|---|---|---|---|---|---|---|---|---|---|
| Working example 6 | E-6 | 1D | 20 | 100 | - | - | 2 A | 400 | 200 | 75 | 13 | 29 | 10.5 | 3 | - |
| Comparative example 11 | c- 11 | 1 D | 20 | 100 | - | - | 2 D | 800 | - | 150 | 12 | 28 | 10.6 | 7.2 | - |
| Comparative example 12 | C- 12 | 1 D | 20 | 100 | - | - | 2 E | - | 400 | 150 | 14 | 29 | 10.4 | 10.4 | - |

Similarly, as can be seen from the results of the working example 6 and comparative examples 11 and 12, even in the cases of the binder-containing photocatalyst thin films and under UV irradiation, the photocatalyst titanium oxide prepared by adding the solution (2A) containing the iron, titanium and silicon components to the titanium oxide particles (1D) exhibited a more improved photocatalytic activity as compared to the photocatalyst titanium oxide prepared by adding the solution (2D) containing the iron and silicon components to the titanium oxide particles, and the photocatalyst titanium oxide prepared by adding the solution (2E) containing the titanium and silicon components to the titanium oxide particles.

The titanium oxide particle dispersion liquid of the present invention is useful for forming a photocatalyst thin film on various members including inorganic substances such as glass and metals; and organic substances such as a resin, when applied thereto. The titanium oxide particle dispersion liquid of the present invention is especially useful for forming a transparent photocatalyst thin film on various members.

**Claims**

1. Titanium oxide particles with

   (1) a tin component and a visible light activity-enhancing transition metal component being solid-dissolved in the particles; and with
   (2) an iron component, a titanium component and a silicon component being adhered to the surfaces of the particles.

2. The titanium oxide particles according to claim 1, wherein the visible light activity-enhancing transition metal component solid-dissolved in the titanium oxide particles is at least one selected from vanadium, chromium, manganese, niobium, molybdenum, rhodium, tungsten and cerium.

3. The titanium oxide particles according to claim 2, wherein the visible light activity-enhancing transition metal component solid-dissolved in the titanium oxide particles is at least one selected from a molybdenum component, a tungsten component and a vanadium component.

4. The titanium oxide particles according to any one of claims 1 to 3, wherein the tin component is contained and solid-dissolved in the titanium oxide particles by an amount of 1 to 1,000 in terms of a molar ratio to titanium oxide ($TiO_2/Sn$).

5. The titanium oxide particles according to any one of claims 1 to 4, wherein a molar ratio of the iron component to titanium oxide ($TiO_2/Fe$) is 10 to 10,000, a molar ratio of the titanium component to titanium oxide ($TiO_2/Ti$) is 10 to 10,000, and a molar ratio of the silicon component to titanium oxide ($TiO_2/Si$) is 1 to 10,000.

6. The titanium oxide particles according to claim 3, wherein the molybdenum, tungsten and vanadium components solid-dissolved in the titanium oxide particles are each in an amount of 1 to 10,000 in terms of a molar ratio to titanium oxide ($TiO_2/Mo$, $TiO_2/W$ or $TiO_2/V$).

7. A titanium oxide particle dispersion liquid with the titanium oxide particles according to any one of claims 1 to 6 being dispersed in an aqueous dispersion medium.

8. The titanium oxide particle dispersion liquid according to claim 7, wherein the titanium oxide particle dispersion liquid further comprises a binder.

9. The titanium oxide particle dispersion liquid according to claim 8, wherein the binder is a silicon compound-based binder.

10. A photocatalyst thin film comprising the titanium oxide particles according to any one of claims 1 to 6.

11. The photocatalyst thin film according to claim 10, wherein the photocatalyst thin film further comprises a binder.

12. A member having, on a surface thereof, the photocatalyst thin film according to claim 10 or 11.

13. A method for producing the titanium oxide particle dispersion liquid according to any one of claims 7 to 9, comprising:

(1) a step of producing a tin component and transition metal component-containing peroxotitanic acid solution from a raw material titanium compound, tin compound, transition metal compound, basic substance, hydrogen peroxide and aqueous dispersion medium;

(2) a step of obtaining a tin component and transition metal component-containing titanium oxide particle dispersion liquid by heating the tin component and transition metal component-containing peroxotitanic acid solution produced in the step (1) at 80 to 250°C under a controlled pressure;

(3) a step of producing a solution or dispersion liquid of the iron component, titanium component and silicon component from an iron compound, titanium compound, silicon compound and aqueous dispersion medium; and

(4) a step of obtaining a dispersion liquid by mixing the titanium oxide particle dispersion liquid that has been produced in the step (2) and the solution or dispersion liquid of the iron, titanium and silicon compounds that has been produced in the step (3).

## INTERNATIONAL SEARCH REPORT

| International application No. |
|---|
| **PCT/JP2021/032391** |

### A. CLASSIFICATION OF SUBJECT MATTER

*B01J 35/02*(2006.01)i; *A61L 9/00*(2006.01)i; *A61L 9/01*(2006.01)i; *B01J 21/06*(2006.01)i; *B01J 23/847*(2006.01)i;
*B01J 23/887*(2006.01)i; *B01J 23/888*(2006.01)i; *B01J 37/04*(2006.01)i; *B01J 37/10*(2006.01)i
FI:   B01J35/02 J; A61L9/00 C; A61L9/01 B; B01J21/06 M; B01J23/847 M; B01J23/887 M; B01J23/888 M; B01J37/04 102;
B01J37/10

According to International Patent Classification (IPC) or to both national classification and IPC

### B. FIELDS SEARCHED

Minimum documentation searched (classification system followed by classification symbols)

B01J35/02; A61L9/00; A61L9/01; B01J21/06; B01J23/847; B01J23/887; B01J23/888; B01J37/04; B01J37/10

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2020-142936 A (SHINETSU CHEMICAL CO) 10 September 2020 (2020-09-10) claims 1-5, paragraphs [0045]-[0072], [0079]-[0080] | 1-4, 6-12 |
| A | WO 2016/152487 A1 (SHIN-ETSU CHEMICAL CO LTD) 29 September 2016 (2016-09-29) claims 1-12 | 1-13 |
| A | JP 2015-007240 A (ENVONT LLC) 15 January 2015 (2015-01-15) entire text, all drawings | 1-13 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **14 October 2021** | **02 November 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2021/032391**

| Patent document cited in search report | | | Publication date (day/month/year) | Patent family member(s) | | | Publication date (day/month/year) |
|---|---|---|---|---|---|---|---|
| JP | 2020-142936 | A | 10 September 2020 | (Family: none) | | | |
| WO | 2016/152487 | A1 | 29 September 2016 | US | 2018/0117567 | A1 | |
| | | | | claims 1-12 | | | |
| | | | | EP | 3275536 | A1 | |
| | | | | CN | 107427818 | A | |
| | | | | KR | 10-2017-0131505 | A | |
| JP | 2015-007240 | A | 15 January 2015 | US | 2009/0162560 | A1 | |
| | | | | entire text, all drawings | | | |
| | | | | WO | 2009/086193 | A2 | |
| | | | | EP | 2231799 | A2 | |
| | | | | KR | 10-2010-0099741 | A | |
| | | | | CN | 101945964 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009148700 A
- JP 2012210632 A
- JP 2010104913 A
- JP 2011240247 A
- JP HEI7303835 A
- WO 2014045861 A
- WO 2016152487 A